# EUROPEAN PATENT SPECIFICATION

(11) **EP 3 650 026 B1**
(45) Date of publication and mention of the grant of the patent: **01.10.2025**
(21) Application number: 18828466.5
(22) Date of filing: 05.07.2018
(51) Int. Cl.: A61K 31/549, A61K 31/548, A61P 9/12

(54) **EPIDITHIODIOXOPIPERAZINE COMPOUND OR DERIVATIVE THEREOF, OR PHARMACEUTICAL COMPOSITION FOR PREVENTING OR TREATING PULMONARY HYPERTENSION, CONTAINING PHARMACEUTICALLY ACCEPTABLE SALTS THEREOF**
EPIDITHIODIOXOPIPERAZIN-VERBINDUNG ODER DERIVAT DAVON ODER PHARMAZEUTISCHE ZUSAMMENSETZUNG ZUR PRÄVENTION ODER BEHANDLUNG VON PULMONALER HYPERTONIE MIT PHARMAZEUTISCH UNBEDENKLICHEN SALZEN DAVON
COMPOSÉ OU DÉRIVÉ D'ÉPIDITHIODIOXOPIPÉRAZINE, OU COMPOSITION PHARMACEUTIQUE DE PRÉVENTION OU DE TRAITEMENT DE L'HYPERTENSION PULMONAIRE, CONTENANT DES SELS PHARMACEUTIQUEMENT ACCEPTABLES DE CE DERNIER

(30) Priority: 05.07.2017 KR 20170085656; 05.01.2018 KR 20180001889
(43) Date of publication of application: 13.05.2020
(73) Proprietor: VASTHERA Co. Ltd., Seoul 03760 (KR)
(72) Inventor: KANG, Sang Won, Seoul 06517 (KR); KWON, Kihwan, Seoul 06009 (KR)
(74) Representative: Ström & Gulliksson AB
(86) International application number: PCT/KR2018/007649
(87) International publication number: WO 2019/009646

(56) References cited:
- EP-A1- 0 926 242
- WO-A1-2011/159706
- WO-A1-2013/077709
- WO-A1-2014/189343
- WO-A2-2006/135949
- KR-A- 20070 102 492
- KR-A- 20140 138 545
- US-A1- 2013 098 357
- US-A1- 2015 246 933
- US-A1- 2016 222 031

## Description

### [Technical Field]

The present invention relates to a pharmaceutical composition for use in the prevention or treatment of pulmonary arterial hypertension, comprising an epidithiodioxopiperazine (ETP) compound or a pharmaceutically acceptable salt thereof, which has an intramolecular disulfide bond in an epidithiodioxopiperazine ring.

### [Background Art]

The pulmonary artery is an artery that carries blood in the body from the right ventricle to the lungs in order to supply oxygen, and pulmonary arterial hypertension is defined as when the mean pulmonary artery pressure at rest is 25 mmHg or greater or when the mean pulmonary artery pressure during exercise is 30 mmHg. Pulmonary arterial hypertension is classified into "primary pulmonary arterial hypertension", which is the case where no specific cause has been disclosed, and specific disease "associated pulmonary arterial hypertension", which occurs secondarily due to a specific causative disease. Examples of the latter case include familial pulmonary arterial hypertension related to heredity, and the specific disease-associated pulmonary arterial hypertension may be caused by collagen vascular diseases (*e.g.,* systemic sclerosis, systemic lupus erythematosus, *etc*.)*,* portal hypertension, HIV infection, congenital heart diseases, and drugs or toxins such as an appetite suppressant or cocaine. Specific disease-associated pulmonary arterial hypertension is not significantly different from primary pulmonary arterial hypertension in terms of spontaneous progression, histopathological findings, response to treatment, *etc.* Primary pulmonary arterial hypertension is 1.7 times more common in women than in men and may occur in all ages, but it is frequent in those in their 20s and 30s. Approximately 7% of patients have a family history of mutation in the gene called *BMPR2,* by which the autosomal dominant trait appears to be inherited. Patients with pulmonary arterial hypertension may present with symptoms such as shortness of breath (dyspnea), fainting, dizziness, peripheral edema (*e.g.,* lower extremity edema), impotent feeling, anepithymia, increased heart rate, headache, precordialgia, cyanoderma, *etc.* Further, it may be accompanied by Raynaud's phenomenon, in which the fingers and toes easily become cold and turn blue. Patients with pulmonary arterial hypertension are often initially exposed to misdiagnosis such as influences from asthma or excessive stress, resulting in breathing difficulties; and after an average of 2.5 years, the patients are diagnosed correctly.

For the treatment of pulmonary arterial hypertension, surgical intervention such as interventional atrial septostomy, lung transplantation, heart-lung transplantation, *etc.* can be performed, but drug treatment is preferred because of the high cost and risk of surgical intervention. Currently, there are methods to use a general vasodilator as a drug for treating pulmonary arterial hypertension, but most vasodilators are calcium channel blockers, which are difficult to apply because they often do not show a significant effect on pulmonary arterial hypertension and have side effects associated with administration. Therefore, it is necessary to discover an effective therapeutic agent for pulmonary arterial hypertension.

US2016222031 and WO2014189343 relate to an epidithiodioxopiperazine derivative having improved intracellular permeability and mimicking the activity of 2-Cys-Prx in its reduced form in the cells as well as a pharmaceutical composition for preventing or treating vascular diseases.

WO2013077709 discloses epidithiodioxopiperazine compounds and their use in hypertension. The pidithiodioxopiperazine compounds are characterized by mimicking 2-Cys-Prx activity. WO201159706 discloses ranolazine for use in the treatment of pulmonary arterial hypertension.

### [Technical Problem]

The present inventors have confirmed that an epidithiodioxopiperazine compound represented by Formula 1, which has an intramolecular disulfide bond in an epidithiodioxopiperazine ring, alleviates the symptoms of pulmonary arterial hypertension in an experimental animal model, thereby completing the present invention, 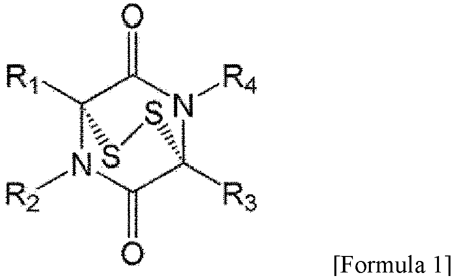 wherein, in Formula 1,
R₁ to R₄ are each independently hydrogen, linear or branched C1 to C6 alkyl, akenyl, or alkynyl, or benzyl substituted with C1 to C6 alkoxy.

### [Technical Solution]

The present invention provides a pharmaceutical composition for use in the prevention or treatment of pulmonary arterial hypertension, comprising an epidithiodioxopiperazine compound represented by Formula 1(see above), or a pharmaceutically acceptable salt thereof, which has an intramolecular disulfide bond in an epidithiodioxopiperazine ring.

### [Advantageous Effects]

The pharmaceutical composition of the present invention, which comprises an epidithiodioxopiperazine (ETP) compound represented by Formula 1(see above), or a pharmaceutically acceptable salt thereof, which has an intramolecular disulfide bond in an epidithiodioxopiperazine ring, can prevent or treat pulmonary arterial hypertension by effectively blocking thickening of the pulmonary arterial wall in a rat model in which pulmonary arterial hypertension is induced by MCT, and thus can prevent the heart, especially the right ventricle, from becoming hypertrophied.

### [Summary]

The present disclosure relates to a pharmaceutical composition for use in the prevention or treatment of pulmonary arterial hypertension, comprising an epidithiodioxopiperazine compound represented by the following Formula 1 or a pharmaceutically acceptable salt thereof, which has an intramolecular disulfide bond in an epidithiodioxopiperazine ring, 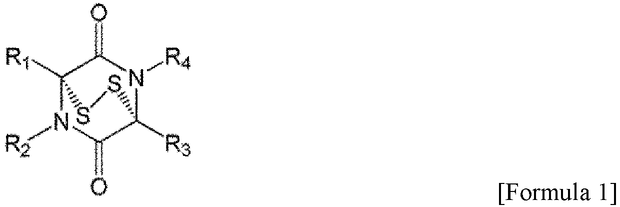 wherein, in Formula 1,
R₁ to R₄ are each independently hydrogen, linear or branched C1 to C6 alkyl, alkenyl, or alkynyl, or benzyl substituted with C1 to C6 alkoxy.

According to one embodiment, R₁ to R₄ are each independently hydrogen, methyl, butyl, propenyl, allyl, methoxybenzyl, methoxypropyl, or benzhydryl.

According to a anther embodiment, the compound represented by Formula 1 is any one of the following Formulas 2 to 7.

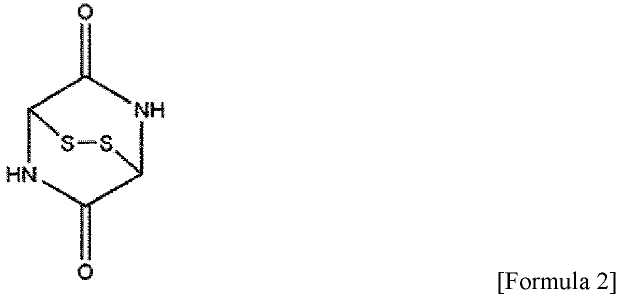

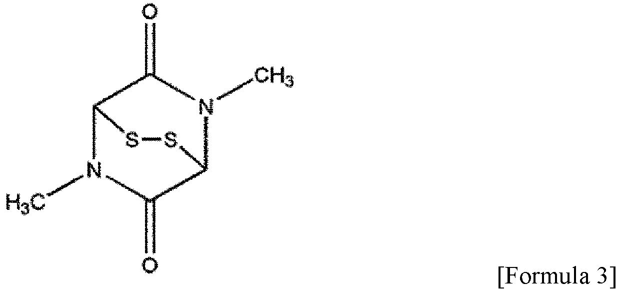

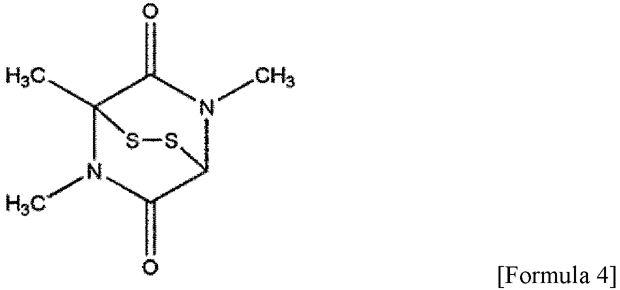

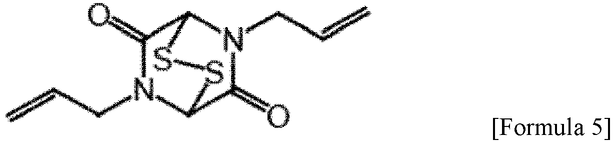

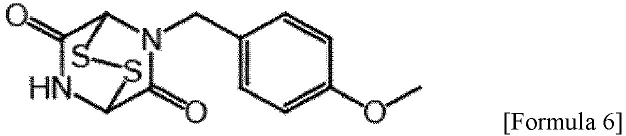

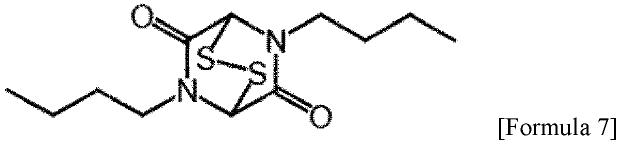

According to a further embodiment, the prevention or treatment of pulmonary arterial hypertension is achieved by mimicking an intracellular activity of PrxII.

### [Brief Description of Drawings]

Fig. 1 is an enlarged view showing the results of H&E staining of hearts excised from the control group, monocrotaline (MCT) administration group, and experimental group administered with 5,7-dimethyl-2,3-dithia-5,7-diazabicyclo[2.2.2]octane-6,8-dione (A-2) after MCT administration.
Fig. 2 is a graph comparing the mass ratio of right ventricles over the sum of left ventricles and cardiac septa in hearts excised from the control group, MCT administration group, and experimental groups administered with dose-dependent A-2 after MCT administration.
Fig. 3 is a graph comparing the average thickness of pulmonary arterial walls excised from the control group, MCT administration group, and experimental groups administered with dose-dependent A-2 after MCT administration.
Fig. 4 is a diagram comparing the morphology and size of the entire hearts and right ventricles excised from the control group, MCT administration group, and experimental groups administered with 2,3-dithia-5,7-diazabicyclo[2.2.2]octane-6,8-dione (A-1), 4,5,7-trimethyl-2,3-dithia-5,7-diazabicyclo[2.2.2]octane-6,8-dione (A-3), and 7-(4-methoxybenzyl)-2,3-dithia-5,7-diazabicyclo[2.2.2]octane-6,8-dione (A-6), respectively, after MCT administration.
Fig. 5 show graphs comparing the total mass of hearts (left) and the mass ratio of right ventricles over the sum of left ventricles and cardiac septa in hearts (right) excised from the control group, MCT administration group, and experimental groups administered with 6 µg of A-1, A-3, and A-6, respectively, after MCT administration.
Fig. 6 is an enlarged view showing the results of H&E staining of hearts excised from the control group, MCT administration group, and experimental groups administered with 6 µg of A-1, A-3, 5,7-diallyl-2,3-dithia-5,7-diazabicyclo[2.2.2]octane-6,8-dione (A-5), and A-6, respectively, after MCT administration.
Fig. 7 is a graph comparing the average thickness of pulmonary arterial walls excised from the control group, MCT administration group, and experimental groups administered with 6 µg of A-1, A-3, A-5, and A-6, respectively, after MCT administration.

### [Best Mode for Carrying Out the Invention]

The present invention provides a pharmaceutical composition for use in the prevention or treatment of pulmonary arterial hypertension, comprising an epidithiodioxopiperazine compound represented by the following Formula 1 or a pharmaceutically acceptable salt thereof, which has an intramolecular disulfide bond in an epidithiodioxopiperazine ring: 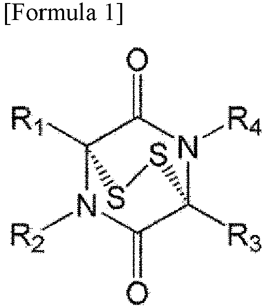 wherein, in Formula 1,
R₁ to R₄ are each independently hydrogen, linear or branched C1 to C6 alkyl, akenyl, or alkynyl, or benzyl substituted with C1 to C6 alkoxy.

Specifically, R₁ to R₄ may each independently be hydrogen, methyl, butyl, propenyl, allyl, methoxybenzyl, methoxypropyl, or benzhydryl.

The present invention is based on the discovery that a series of synthetic small molecule compounds having an intramolecular disulfide bond in an epidithiodioxopiperazine ring exerts an effect of treating pulmonary arterial hypertension through the intramolecular disulfide bond included therein. Specifically, the present inventors have found that 2,3-dithia-5,7-diazabicyclo[2.2.2]octane-6,8-dione, 5,7-dimethyl-2,3-dithia-5,7-diazabicyclo[2.2.2]octane-6,8-dione, 4,5,7-trimethyl-2,3-dithia-5,7-diazabicyclo[2.2.2]octane-6,8-dione, and 7-(4-methoxybenzyl)-2,3-dithia-5,7-diazabicyclo[2.2.2]octane-6,8-dione, which are small molecule epidithiodioxopiperazine derivatives synthesized to identify a key mechanism of action involved in treating the pulmonary arterial hypertension, showed an excellent therapeutic effect on pulmonary arterial hypertension; whereas, the present inventors have also found that a reduced derivative, which similarly includes a dioxopiperazine ring and has two thiol groups instead of the intramolecular disulfide bond, does not exhibit a therapeutic effect on pulmonary arterial hypertension. Therefore, it is obvious that an epidithiodioxopiperazine derivative including an intramolecular disulfide bond can exert an effect of treating pulmonary arterial hypertension regardless of the type of substituent.

The pulmonary arterial hypertension is a type of hypertension that affects the artery in the lungs and the right side of the heart, and is defined when the mean pulmonary artery pressure at rest is 25 mmHg or greater or when the mean pulmonary artery pressure during exercise is 30 mmHg.

In one form of pulmonary arterial hypertension, small arteries (*i.e.,* pulmonary arteries) and capillaries in the lungs may become narrow, clogged, or damaged, which makes blood flow through the lung harder and increases the pressure inside the pulmonary artery. As a result of the increased pressure, it makes it difficult for the lower right chamber (right ventricle) to pump the blood through the lung, eventually weakening the heart muscles to cause the loss of its function.

Other forms of pulmonary arterial hypertension can continuously be exacerbated, and may sometimes be fatal and serious conditions. Even if some form of pulmonary arterial hypertension cannot be recovered completely, the treatment therefor may help alleviate its symptoms and improve quality of life.

Drugs for specifically treating the pulmonary arterial hypertension have not yet been discovered, and thus conventional vasodilators such as short-acting vasodilators or calcium channel blockers are used for the drug therapy for the pulmonary arterial hypertension. However, cases in which patients respond significantly to short-acting vasodilators are very few, less than about 10% of patients with diagnosis, and there are also cases where patients do not respond to calcium channel blockers. In addition, even if the responses occur, administration is limited due to the numerous side effects of the drugs. Accordingly, pulmonary arterial hypertension differs from general vascular diseases in its treatment mechanism, so that the types of drugs that can be applied are very limited. Further, drugs that have an effect on vascular diseases cannot be expected to have a therapeutic effect on pulmonary arterial hypertension.

The epidithiodioxopiperazine compound refers to a compound including an epidithiodioxopiperazine ring, that is, the structural nucleus showing an activity. The compound may include a compound in which the NH group or the CH group in the ring of the compound represented by Formula 2 is substituted as stated above for R₁ to R₄ The modification and substitution of the structure of the compound of Formula 2 can be easily carried out by those skilled in the art, for example, as follows:

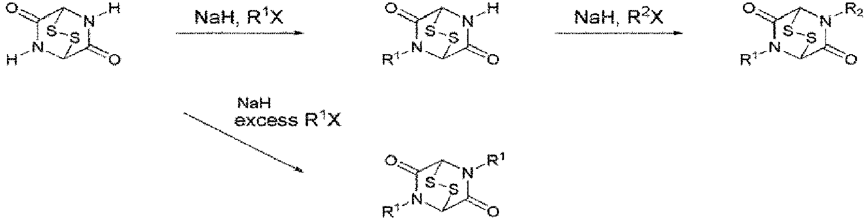

For example, the the epidithiodioxopiperazine compound may be any one of the compounds represented by the following Formulas 2 to 7.

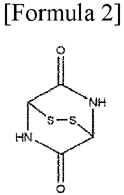

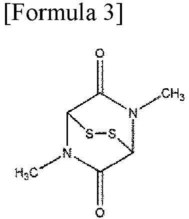

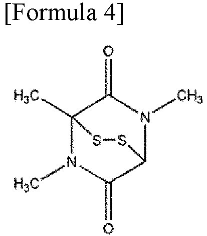

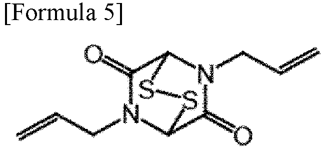

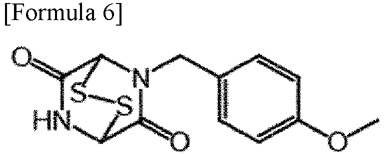

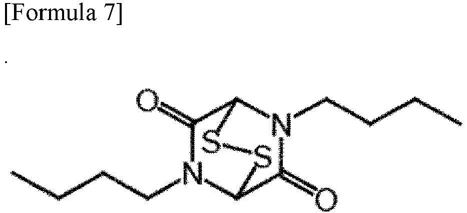

The compounds represented by Formulas 2 to 7 can be synthesized and used by those skilled in the art by referring to known methods. As a specific synthesis method, reference can be made to the method disclosed in Korean Patent No. 1633975.

In an exemplary embodiment of the present invention, among a series of the epidithiodioxopiperazines synthesized as described above, which include an intramolecular disulfide bond, 2,3-dithia-5,7-diazabicyclo[2.2.2]octane-6,8-dione (Formula 2), 5,7-dimethyl-2,3-dithia-5,7-diazabicyclo[2.2.2]octane-6,8-dione (Formula 3), 4,5,7-trimethyl-2,3-dithia-5,7-diazabicyclo[2.2.2]octane-6,8-dione (Formula 4), 5,7-diallyl-2,3-dithia-5,7-diazabicyclo[2.2.2]octane-6,8-dione (Formula 5), and 7-(4-methoxybenzyl)-2,3-dithia-5,7-diazabicyclo[2.2.2]octane-6,8-dione (Formula 6) was confirmed to have an effect of treating pulmonary arterial hypertension. Specifically, it was confirmed that when the compounds of Formulas 2 to 6 were administered to experimental animals, in which pulmonary arterial hypertension had been induced by administering monocrotaline, the remarkable hypertrophy of the right ventricle and pulmonary artery intimal thickening, which are shown in a pulmonary arterial hypertension model, were reduced to a level similar to normal. This result is an effect achieved through the intramolecular disulfide bond, and therefore, it is apparent to those skilled in the art that the compound of Formula 7, which includes the intramolecular disulfide bond, shares the same structural nucleus as the compound of Formulas 2 to 6, and in which only the nitrogen atoms on the ring are substituted with substituents having a similar property, will also have an equivalent effect.

The epidithiodioxopiperazine compound may be separated from natural sources, acquired from natural sources and then prepared by chemical reforming, or prepared from chemical synthesis by those skilled in the art referencing known preparation methods. Preferably, the epidithiodioxopiperazine compound may be used by being separated from bacteria, a culture medium thereof, or metabolites according to known methods in the art, or prepared from syntheses using methods described in the prior patent of the present invention (Korean Patent No. 1633975).

The composition of the present invention may achieve an effect of preventing or treating pulmonary arterial hypertension by mimicking an intracellular PrxII activity.

The epidithiodioxopiperazine compound of the present invention may be used in the form of a pharmaceutically acceptable salt. In addition, the compound of the present invention may be used alone or in combination with other pharmaceutically acceptable compounds.

The term "pharmaceutically acceptable salt" used in the present invention refers to all salts having target biological and/or physiological activities of the compound, and minimally exhibiting undesirable toxicological effects. In the present invention, the type of the salt is not limited as long as the salt maintains a diketopiperazine ring including an intramolecular disulfide bridge. As the salt, an acid addition salt formed by a pharmaceutically acceptable free acid is useful. The acid addition salt may be prepared using common methods such as dissolving a compound in an excess aqueous solution, and precipitating this salt using a water-miscible organic solvent such as methanol, ethanol, acetone, or acetonitrile. An equimolar compound, and an acid or alcohol in water (for example, glycol monomethyl ether) are heated, and then the mixture may be dried by evaporation, or the precipitated salt may be suction filtered. Herein, an inorganic acid or an organic acid may be used as the free acid, and hydrochloric acid, hydrobromic acid, phosphoric acid, nitric acid, sulfuric acid, stannic acid, and the like may be used as the inorganic acid, and methanesulfonic acid, *p*-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, and the like may be used as the organic acid.

In addition, a pharmaceutically acceptable metal salt may be prepared using a base. An alkali metal or alkaline earth metal salt is obtained by, for example, dissolving a compound in an excess alkali metal hydroxide or alkaline earth metal hydroxide solution, filtering the non-soluble compound salt, drying the filtrate, and drying the result. Herein, preparing a sodium, potassium, or calcium salt as the metal salt is pharmaceutically suitable. Furthermore, a corresponding silver salt may be obtained by reacting the alkali metal or alkaline earth metal salt with a suitable silver salt (for example, silver nitrate).

The pharmaceutically acceptable salt of the epidithiodioxopiperazine compound according to the present invention includes, unless otherwise specified, all salts of acidic or basic groups that can exist. For example, the pharmaceutically acceptable salt may include sodium, calcium, and potassium salts of a hydroxyl group, and as other pharmaceutically acceptable salts of an amino group, hydrobromide, sulfate, hydrogen sulfate, phosphate, hydrogen phosphate, dihydrogen phosphate, acetate, succinate, citrate, tartrate, lactate, mandelate, methanesulfonate (mesylate), p-toluenesulfonate (tosylate) salts, and the like may be included, and these may be prepared using preparation methods of salts known in the art.

The composition according to the present invention may further include proper carriers, diluting agents, and diluents commonly used for the preparation of pharmaceutical compositions. The composition is sterilized or aseptic, may be water, a buffer, an isotonic agent, and the like, and the solution is sterilized or aseptic, or may include other ingredients known to those skilled in the art, which do not cause allergies or other harmful reactions when applied to animals or humans.

The term "pharmaceutically acceptable carrier" used in the present invention includes all random solvents, dispersive media, coating materials, antimicrobial agents, antifungal agents, isotonic agents, and the like. Using the media and the formulations as pharmaceutically active materials is well known in the related art. In addition to common media or formulations non-miscible with active ingredients, the use of the media and the formulations described above is considered in therapeutic compositions. In addition, supplementary active ingredients may be mixed with the composition described above.

The composition may be prepared as formulations such as liquids, emulsions, suspensions, or creams, or may be used for parenteral administration. The amount of the composition used may be an amount commonly used for preventing vascular restenosis, and is preferably different depending on the age, gender, and condition of patients, *in vivo* absorbance of active substances, inactivation rate, and drugs used in combination.

Prevention or treatment of pulmonary arterial hypertension comprises administering the pharmaceutical composition to a subject in need thereof.

In the present invention, the term "prevention" refers to all actions that suppress pulmonary arterial hypertension or delay the outbreak of the diseases by the administration of the pharmaceutical composition, and the term "treatment" refers to all actions that enable the symptoms of pulmonary arterial hypertension to improve or change for the better by the administration of the pharmaceutical composition.

Herein, the term "subject" refers to all animals including human beings which have developed or have a possibility of developing pulmonary arterial hypertension, and the pulmonary arterial hypertension may be effectively prevented or treated by administering the pharmaceutical composition of the present invention into a subject. In addition, the pharmaceutical composition of the present invention may be administered in combination with known therapeutic agents for pulmonary arterial hypertension.

The pharmaceutical composition of the present invention is administered with a therapeutically effective dose. The term "therapeutically effective dose" refers to an amount sufficient to treat diseases in a reasonable benefit/risk ratio applicable to medical treatments and not to cause side effects, and the level of the effective dose may be readily determined by those skilled in the art depending on the factors including the gender, age, weight, and health condition of patients, severity of the disease, the activity of drugs, the sensitivity to drugs, administration methods, administration time, administration routes and excretion rates, treatment period, drugs mixed or simultaneously used, and other factors well known in the field of medicine.

The term "administration" as used herein refers to introducing a prescribed material to a patient using proper methods, and the composition may be administered via any general route as long as the composition reaches a target tissue. The administration method can be oral or parenteral administration by formulating the material in the form of a pill, *etc.* In the case of parenteral administration, it can be achieved by intravenous injection or by inhalational administration such as subcutaneous administration or nasal administration. In addition, the prescribed material can be topically administered to lesions, and for topical administration of drugs, double balloon catheters, dispatches or microporous balloons, and the like may be used, and particularly, stents or sustained microparticles may be used for long-term drug delivery. Most preferably, the composition of the present invention may be directly administered to the area of occurrence of pulmonary arterial hypertension by applying the composition inside a stent.

The composition of the present invention can be used for inhalational administration by putting the composition into an injection device in the form of a syringe or spray injection device. For example, it may include an injection device in the form of a tube, through which a drug can be injected into the nasal cavity. Alternatively, in the case where an injection device in the form of a syringe is included, after inserting a discharge part, which corresponds to a syringe needle, into the nasal cavity of a subject, the composition of the present invention which is supported in the form of liquid can be nasally administered by operating a discharge pressure applying part, which corresponds to the piston of the syringe. In particular, the form of the injection device is not limited to the conventional form of a syringe. In the case where an injection device in the form of a spray injection device is included, as in the case of using a syringe form as an injection device, after locating a discharge part in the nostrils which is the entrance of the nasal cavity or in the nasal cavity, the composition of the present invention can be nasally administered in the form of spray by applying an injection pressure. In the case where an injection device in the form of a tube is included, as described above, after inserting a discharge part into the nasal cavity of a subject, the composition of the present invention can be injected into the nasal cavity in the form of liquid by way of applying a pressure to the tube. The above-described injection device forms are merely specific examples, and any device which can inject the composition of the present invention into the nasal cavity can be available.

A drug delivery device for topical administration including the pharmaceutical composition for preventing or treating pulmonary arterial hypertension is described below. The drug delivery device for topical administration may include double balloon catheters, dispatches, microporous balloons, stents, and the like, and is preferably a stent.

The term "stent" in the present invention means a general device for endoluminal application as described above such as intravascular application, and means a cylindrical medical material normalizing a blood flow by being inserted to a narrowed or clogged vascular area under fluoroscopy without surgical laparotomy when the blood flow is disabled due to the development of diseases at a location to have a smooth blood flow. For example, a vascular stent is described in "Textbook of Interventional Cardiology" (Saunders Company, 1994) written by Eric J. Topol. Preferably, the stent is a sustained drug-releasing stent.

As the method of coating the pharmaceutical composition of the present invention onto the stent, common coating methods known to those skilled in the art may be applied, and examples thereof include a dip-coating method and a polymer-coating method, the dip-coating method is the simplest coating method, and biological effects of the drug itself are readily observed since only the pharmaceutical composition is coated. Preferably, the stent of the present invention may be prepared by coating the composition on a drug-releasing stent after being mixed with a polymer material so that the composition according to the present invention is slowly released. The polymer material that can be used as a drug-releasing stent is widely known in the art, and examples thereof include polyurethane, polyethylene terephthalate, PLLA-poly-glycolic acid copolymer (PLGA), polycaprolactone, poly-(hydroxybutyrate/hydroxyvalerate) copolymer, polyvinylpyrrolidone, polytetrafluoroethylene, poly(2-hydroxyethylmethacrylate), poly(ether urethane urea), silicone, acryl, epoxide, polyester, urethane, pyrene, a polyphosphazine polymer, a fluoro polymer, polyamide, polyolefin, and a mixture thereof.

The stent may be formed with one or more materials selected from the group consisting of polysaccharide, heparin, gelatin, collagen, alginate, hyaluronic acid, alginic acid, carrageenan, chondroitin, pectin, chitosan, and derivatives and copolymers thereof, or may be further coated with an antithrombotic layer including these. These materials may be properly combined to a biocompatible topcoat as described in US Patent Application Laid-Open Publication No. US 2006/0083772. The method for forming a stent from the mixture of a polymer and a drug compound is disclosed in Blindt et al., 1999, Int. J. Artif. Organs, 22: 843-853.

### [Mode for Carrying Out the Invention]

Hereinbelow, the present invention will be described in detail with accompanying exemplary embodiments.

### Preparation Example 1: Preparation of monocrotaline-induced pulmonary arterial hypertension experimental animals

Male SD rats weighing approximately 210 g were used as animal models. In order to induce pulmonary arterial hypertension, a single intraperitoneal injection of monocrotaline (MCT) at a dose of 60 mg/kg was carried out. Accordingly, the rat models in which pulmonary arterial hypertension is induced were maintained for 3 weeks while test drugs were administered or not administered, and then the rat models were sacrificed. Thereafter, the hearts were excised and analyzed.

### Example 1: Therapeutic effect 1 of epidithiodioxopiperazine compounds for pulmonary arterial hypertension

5,7-Dimethyl-2,3-dithia-5,7-diazabicyclo[2.2.2]octane-6,8-dione (the compound of Formula 3; hereinafter referred to as "A-2"), which is an epidithiodioxopiperazine compound, was administered to the pulmonary arterial hypertension animal models prepared according to Preparation Example 1 above at each dose of 3 µg and 6 µg every 3 days. As in Preparation Example 1, normal rats in which MCT is not administered were used as the control group; and rats in which pulmonary arterial hypertension is induced by administering only MCT were used as the MCT group. The control group and each experimental group consisted of 3 to 4 rats.

Similar to Preparation Example 1, the rats were maintained for 2 weeks, and then the hearts were excised. The excised hearts were measured for their total mass. Thereafter, the right ventricle was separated and the masses of each of the right ventricle, left ventricle, and cardiac septum were separately measured to calculate the ratio.

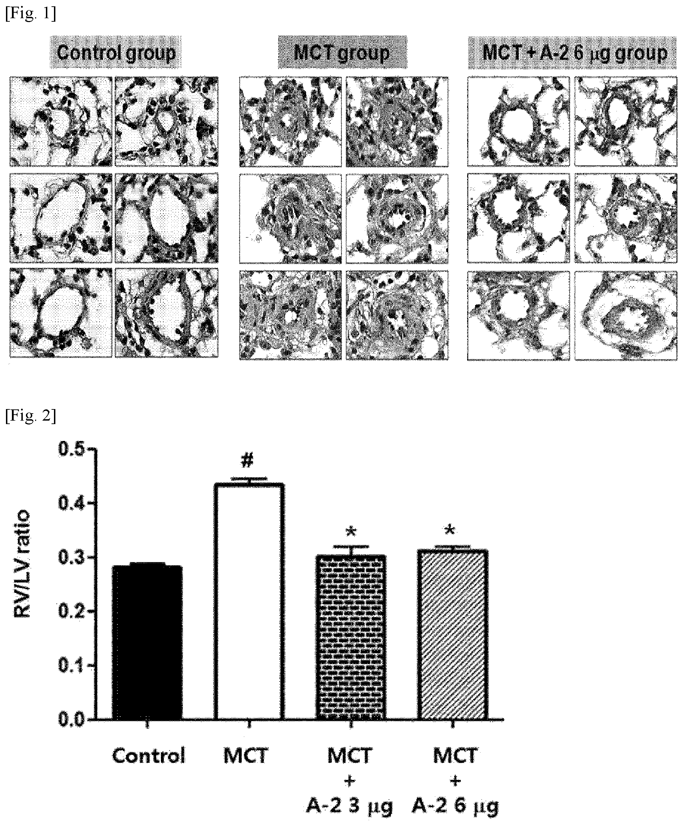

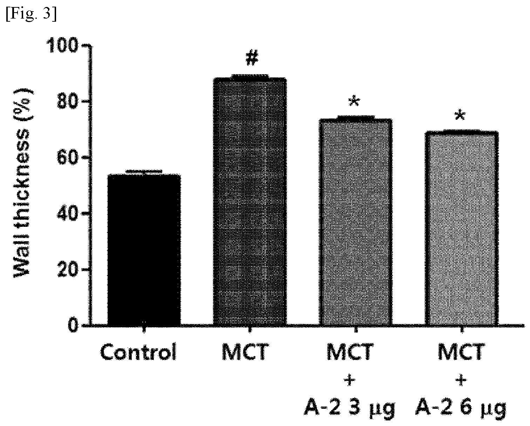

Further, pulmonary artery walls were observed from the excised hearts through H&E staining, and the results thereof are shown in Fig. 1. In addition, in order to visualize the difference in the heart and pulmonary artery according to the dose-dependent A-2 administration after the pulmonary arterial hypertension induction in the pulmonary arterial hypertension model, the average values of the ratio of the right ventricle to the left ventricle and the thickness of the pulmonary artery wall, which are calculated from the heart excised from each animal model, are shown in Figs. 2 and 3, respectively.

As a result, as shown in Fig. 1, the severe thickening of the pulmonary artery wall was observed in the MCT administration group; however, it was confirmed that such thickening of the pulmonary artery wall was relieved to a level similar to the normal level in the group in which A-2 was administered after the MCT administration.

In addition, as shown in Fig. 2, in the MCT administration group, the size of the heart, especially the size of the right ventricle over the left ventricle was increased by about 50%, but in the group in which A-2 was administered after the MCT administration, the size of the right ventricle was recovered to the normal level.

Furthermore, as shown in Fig. 3, in the MCT administration group, the thickness of the artery wall was thickened up to 90%, but in the group administered with A-2 after the MCT administration, the hypertrophied thickness of the artery wall was again reduced to the normal level.

Overall, it was confirmed that the A-2 administration at all doses could effectively block the hypertrophy of the pulmonary artery wall and the hypertrophy of the heart, especially the right ventricle, which are induced by MCT administration.

### Example 2: Therapeutic effect 2 of epidithiodioxopiperazine compounds for pulmonary arterial hypertension

Through Example 1, the efficacy in treating pulmonary arterial hypertension of A-2, one of the epidithiodioxopiperazine derivative compounds, was confirmed, and thus, it was confirmed whether additional epidithiodioxopiperazine derivative compounds have the identical efficacy.

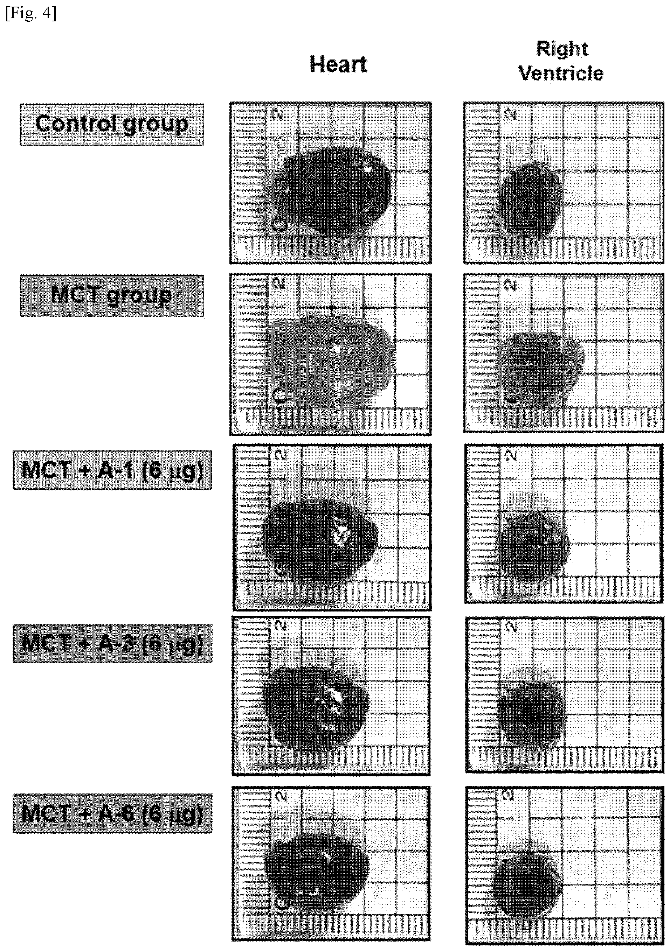

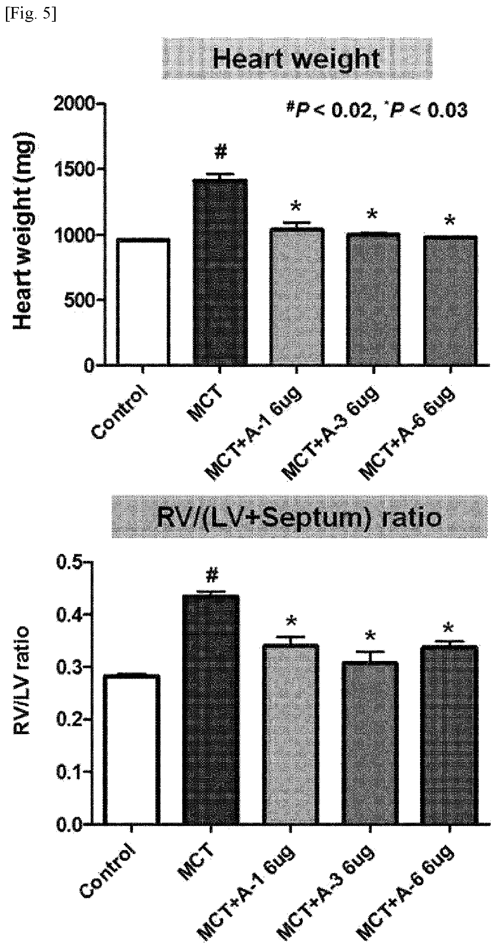

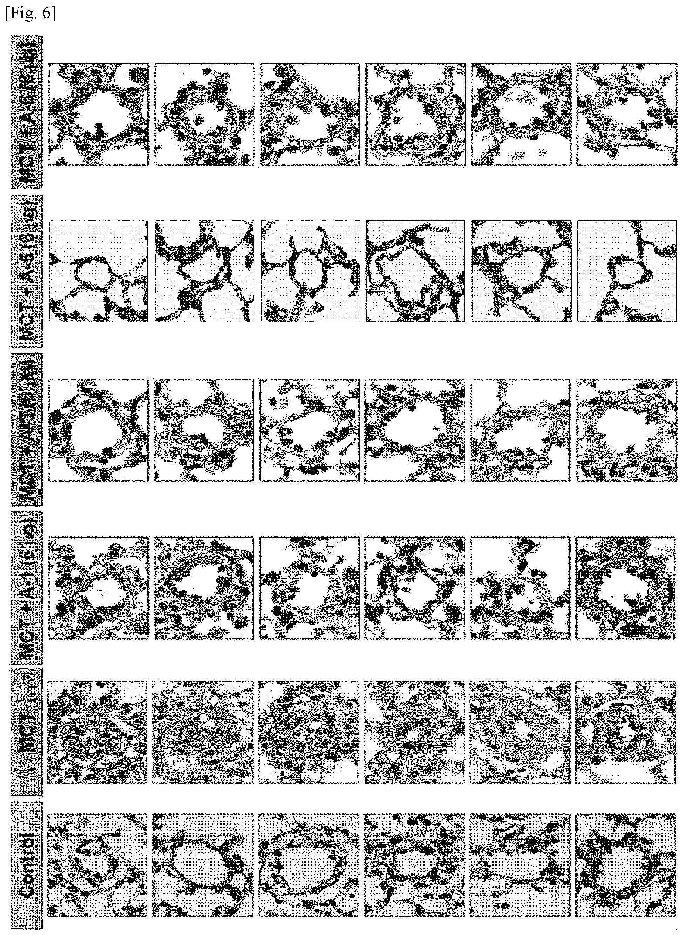

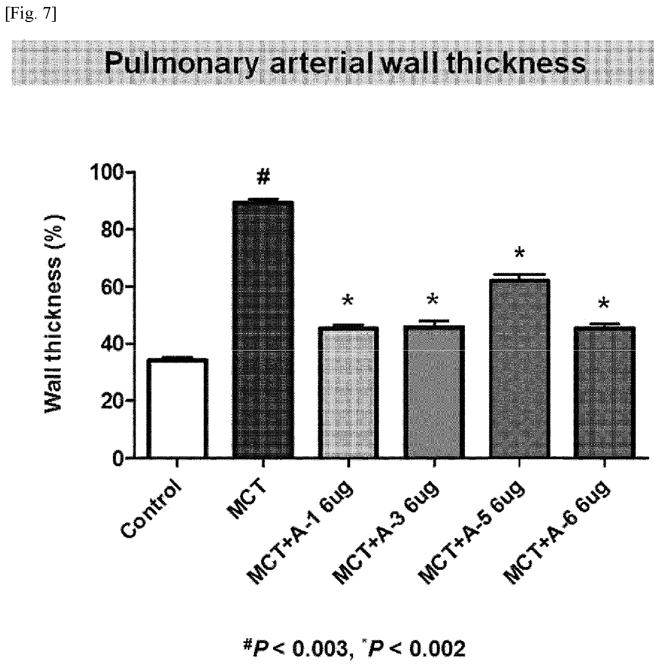

Specifically, except the case of using 2,3-dithia-5,7-diazabicyclo[2.2.2]octane-6,8-dione (the compound of Formula 2; hereinafter referred to as "A-1"), 4,5,7-trimethyl-2,3-dithia-5,7-diazabicyclo[2.2.2]octane-6,8-dione (the compound of Formula 4; hereinafter referred to as "A-3"), 5,7-diallyl-2,3-dithia-5,7-diazabicyclo[2.2.2]octane-6,8-dione (the compound of Formula 5; hereinafter referred to as "A-5"), and 7-(4-methoxybenzyl)-2,3-dithia-5,7-diazabicyclo[2.2.2]octane-6,8-dione (the compound of Formula 6; hereinafter referred to as "A-6") at each dose of 6 µg, instead of A-2, the same method as in Example 1 was performed and the size of the excised entire hearts and separated right ventricles were observed with the naked eye as shown in Fig. 4. Further, the mass of the heart and the ratio of the right ventricle to the left ventricle were measured and shown in Fig. 5. Moreover, the thickness of the pulmonary artery wall was measured through H&E staining of the excised heart fragments and shown in Fig. 6. Therefrom, the average values of the measured thickness of the pulmonary artery were shown in the graph of Fig. 7.

As a result, as shown in Figs. 4 and 5, the size of the hypertrophied heart caused by MCT, especially the size of the right ventricle, was reduced to a level similar to the normal level all by the administration of the epidithiodioxopiperazine derivative compounds, A-1, A-3, and A-6.

Further, as shown in Figs. 6 and 7, the hypertrophy of the pulmonary arterial wall induced by MCT administration was recovered to the normal level all by the administration of A-1, A-3, A-5, and A-6.

Overall, it was confirmed that A-1, A-3, A-5, and A-6, which belong to the epidithiodioxopiperazine compounds according to the invention, exhibit an effect of effectively relieving the symptoms of the pulmonary arterial hypertension.

## Claims

1. A pharmaceutical composition for use in the prevention or treatment of pulmonary arterial hypertension, comprising an epidithiodioxopiperazine compound represented by the following Formula 1 or a pharmaceutically acceptable salt thereof, which has an intramolecular disulfide bond in an epidithiodioxopiperazine ring, 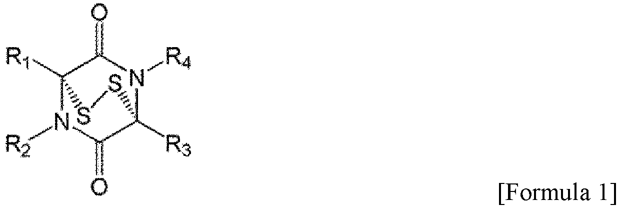 wherein, in Formula 1,
R₁ to R₄ are each independently hydrogen, linear or branched C1 to C6 alkyl, alkenyl, or alkynyl, or benzyl substituted with C1 to C6 alkoxy.

2. The pharmaceutical composition for use according to claim 1, wherein R₁ to R₄ are each independently hydrogen, methyl, butyl, propenyl, allyl, methoxybenzyl, methoxypropyl, or benzhydryl.

3. The pharmaceutical composition for use according to claim 1, wherein the compound represented by Formula 1 is any one of the following Formulas 2 to 7.

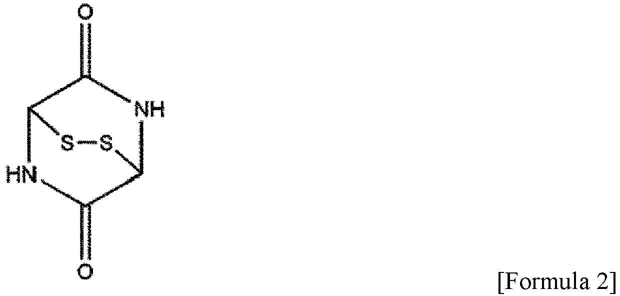

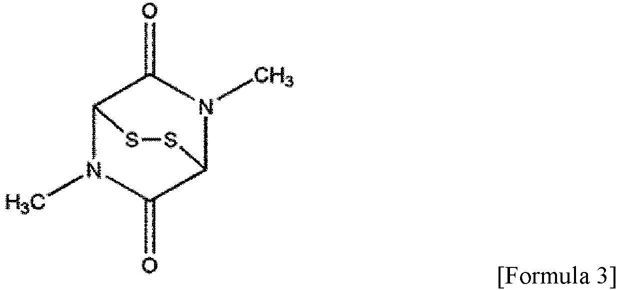

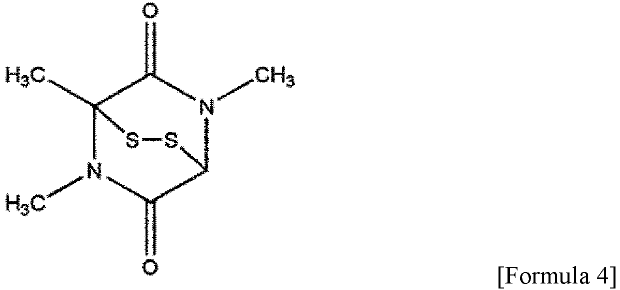

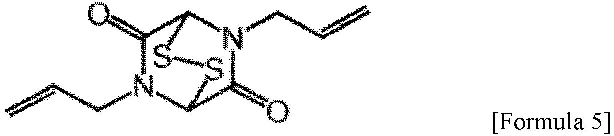

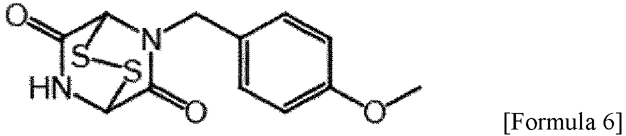

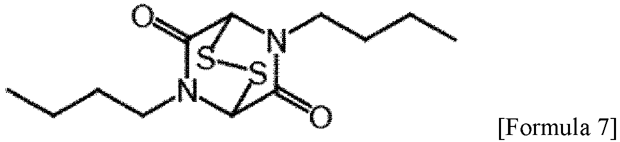

4. The pharmaceutical composition for use according to claim 1, wherein the prevention or treatment of pulmonary arterial hypertension is achieved by mimicking an intracellular activity of PrxII.

## Patentansprüche

1. Pharmazeutische Zusammensetzung zur Verwendung in der Prävention oder Behandlung von pulmonaler arterieller Hypertonie, die eine Epidithiodioxopiperazinverbindung aufweist, die durch die folgende Formel 1 repräsentiert wird, oder ein pharmazeutisch verträgliches Salz davon, die eine intramolekulare Disulfidbindung in einem Epidithiodioxopiperazinring aufweist, 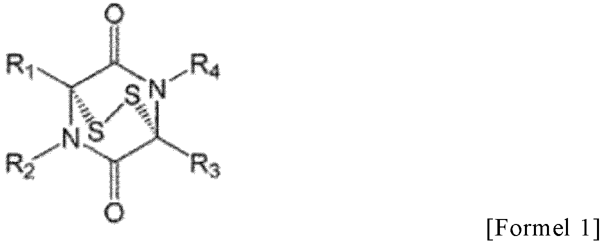 wobei, in der Formel 1,
R₁ bis R₄ jeweils unabhängig voneinander Wasserstoff, lineares oder verzweigtes C1- bis C6-Alkyl, -Alkenyl oder -Alkinyl, oder Benzyl substituiert mit C1- bis C6-Alkoxy, sind.

2. Pharmazeutische Zusammensetzung zur Verwendung gemäß Anspruch 1, wobei R₁ bis R₄ jeweils unabhängig voneinander Wasserstoff, Methyl, Butyl, Propenyl, Allyl, Methoxybenzyl, Methoxypropyl oder Benzhydryl sind.

3. Pharmazeutische Zusammensetzung zur Verwendung gemäß Anspruch 1, wobei die durch Formel 1 repräsentierte Verbindung eine der folgenden Formeln 2 bis 7 ist.

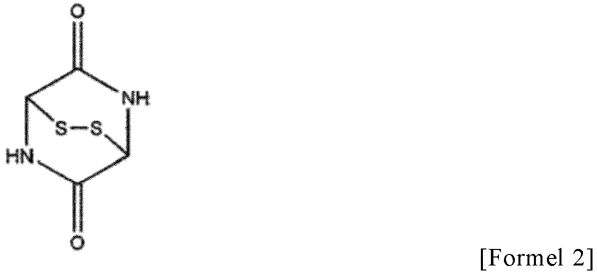

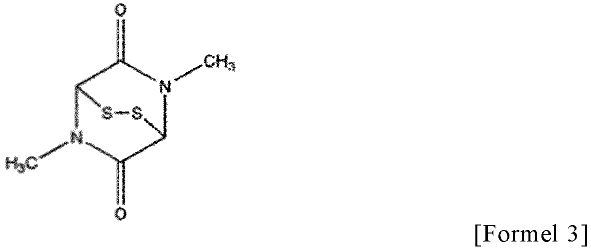

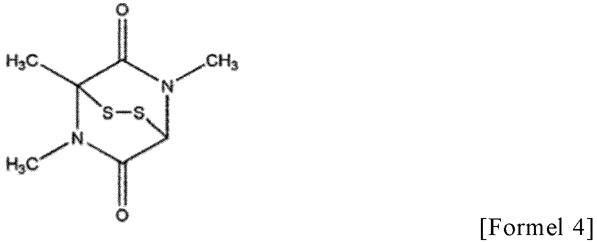

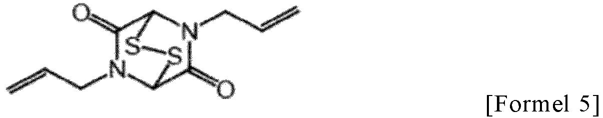

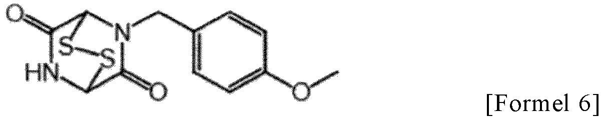

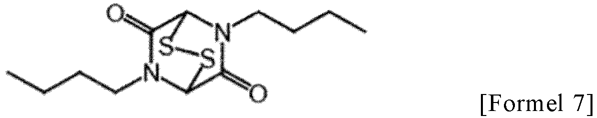

4. Pharmazeutische Zusammensetzung zur Verwendung gemäß Anspruch 1, wobei die Prävention oder Behandlung von pulmonaler arterieller Hypertonie durch Nachahmung einer intrazellulären Aktivität von PrxII erreicht wird.

## Revendications

1. Composition pharmaceutique pour son utilisation dans la prévention ou le traitement de l'hypertension artérielle pulmonaire, comprenant un composé épidithiodioxopipérazine représenté par la formule 1 suivante ou un sel pharmaceutiquement acceptable de celui-ci, qui présente une liaison disulfure intramoléculaire dans un cycle épidithiodioxopipérazine, 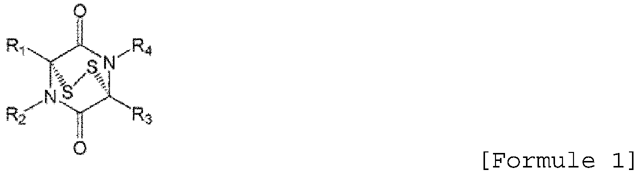 dans laquelle, dans la formule 1,
R₁ à R₄ sont chacun indépendamment hydrogène, alkyle, alcényle ou alkynyle en C1 à C6 linéaire ou ramifié, ou benzyle substitué par alcoxy en C1 à C6.

2. Composition pharmaceutique pour son utilisation selon la revendication 1, dans laquelle R₁ à R₄ sont chacun indépendamment hydrogène, méthyle, butyle, propényle, allyle, méthoxybenzyle, méthoxypropyle, ou benzhydryle.

3. Composition pharmaceutique pour son utilisation selon la revendication 1, dans laquelle le composé représenté par la formule 1 est l'un quelconque des formules 2 à 7 suivantes.

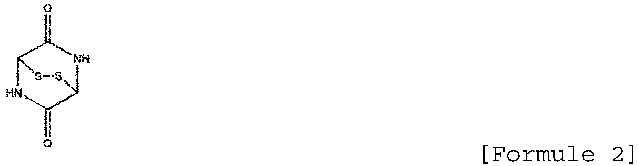

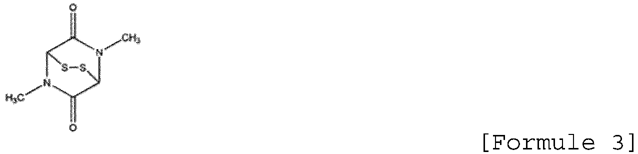

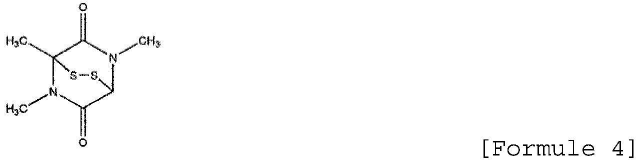

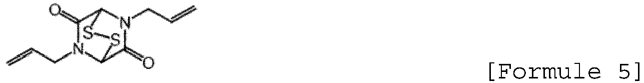

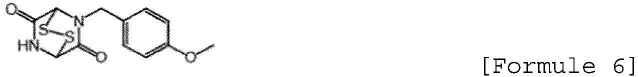

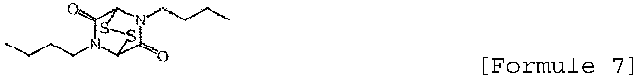

4. Composition pharmaceutique pour son utilisation selon la revendication 1, dans laquelle la prévention ou le traitement de l'hypertension artérielle pulmonaire est obtenu(e) en imitant une activité intracellulaire de PrxII.
